# EUROPEAN PATENT SPECIFICATION

(11) **EP 2 638 874 B1**
(45) Date of publication and mention of the grant of the patent: **29.03.2017**
(21) Application number: 13466005.9
(22) Date of filing: 14.03.2013
(51) Int. Cl.: A61B 17/70

(54) **Transpedicular polyaxial screw**
Transpedikuläre Polyaxialschraube
Vis polyaxiale transpédiculaire

(30) Priority: 16.03.2012 CZ 20120192
(43) Date of publication of application: 18.09.2013
(73) Proprietor: Bertagnoli, Rudolf, 1190 Wien (AT)
(72) Inventor: Jirsák, Václav, 170 00 Praha 7 (CZ); Srámek, Jiri, 170 00 Praha 7 (CZ)
(74) Representative: Skoda, Milan

(56) References cited:
- EP-A1- 1 842 503
- US-A1- 2002 058 942
- US-A1- 2006 235 389
- US-B2- 6 974 460

## Description

### Technical Field

The invention concerns a fixing device to be used in spine surgery.

### State of the Art

At the present time, transpedicular fixation of the spine is a standard and dominant method of posterior fixation of the thoracic and lumbar spine, of sacrum and partly also of the cervical spine using smaller implants and CT navigation. The pedicle is a paired cylinder-shaped anatomic structure that projects from the superodorsolateral part of the vertebral body in the dorsal or slightly dorsolateral direction which is, along with the lamina, part of the vertebral arch. The vertebral arch demarcates the spinal canal with neural structures (spinal cord, nerve roots). The principle of the transpedicular insertion lies in placing the screw using the posterior approach via the pedicle into the vertebral body. The principle of the transpedicular fixation lies in connection of heads of individual screws with a fixing rod. The number of connected segments of the spine is not limited. Basic types of transpedicular screws are so-called polyaxial implants which are based on the connection between a threaded shaft and the head of the screw on the ball journal principle. Some of the first transpedicular screw designs were, for example, those described in patent application No. US 5672176.

This design enables the head of the shaft to be extremely well movable against the shaft. This solution also enables the operating surgeon to place the rod on the head of the screws in a very comfortable way even without any need for accurate shaping of the rod. This means that fixation of more segments is very easy. After the arresting screw is tightened, the fixation rod in the head of the screw presses directly, or via an inserted component, to the spherical end, thus fixing the shaft against the head in the required position. Nowadays, there exists a wide range of small modifications to the design of the polyaxial screws as for their shapes and functions of the arresting screw as well as for the shape and function of the component between the fixation rod and the spherical end of the shaft. In most cases, the head of the polyaxial screw is cylinder-shaped. The diameter of the cylinder is 11 to 15 millimeters and its height is 15 to 20 millimeters. What is common with almost all polyaxial screws is the placing of the spherical end of the shaft in the head of the screw in a symmetrical position where the longitudinal axis of the cylindrical head intersects the centre of the spherical end of the shaft.

Partial exception is the design/solution described in patent application No. US 6974460, where there is a bend approximately in the middle of the head but the spherical end of the shaft is, again, placed symmetrically in such a manner that the longitudinal axis of the lower part of the cylindrical head intersects the centre of the spherical end of the shaft. Moreover, the bend is placed in the plane that is parallel to the axis of the fixation rod which means that it facilitates higher deflection of the threaded shaft against the head of the screw in the sagittal plane but it does not allow the head of the screw to get closer to the vertebra or to move away from it. An alternative solution is described in patent application No. US 20020058942, where the spherical end of the threaded shaft is not rounded by the head of the screw along the whole circumference of the shaft but only along part of it. This solution, again, facilitates higher deflection of the threaded shaft, in the defined sector, against the head of the screw, nevertheless, the spherical end of the shaft is, again, placed symmetrically in such a manner that the longitudinal axis of the cylindrical head intersects the centre of the spherical end of the shaft.

The State of the Art knows the patent application EP 1842503A1, which discloses the preamble of claim 1, and the patent application US 2006/235389 which discloses a polyaxial screw in which the shaft of the screw is offset from its head. In both these designs the spherical end of the shaft is placed symmetrically in the cylindrical head in such a manner that the longitudinal axis of the cylindrical head intersects the centre of the spherical end of the shaft.

All these designs cause the head of the screw to overhang the centre of the spherical end of the shaft in all directions by at least 6 millimeters. If the screw is inserted optimally through the centre of the pedicle in its longitudinal axis (Weinstein's Method), it is necessary to resect the outer rim of the facet joint. If the resection exceeds 3 to 4 millimeters, which is often necessary, irreversible damage to the facet joints occurs in 30 to 40 percent of causes according to recent studies. Former techniques, e.g. the Roy-Camille technique, caused damage to facet joints even in almost 100 percent of causes. This may cause patients permanent postoperative trouble despite optimal insertion of the screws.

### Principle of the Invention

The aforementioned weaknesses are, to a large extent, eliminated and the goals of the invention accomplished by a transpedicular polyaxial screw with an eccentrically positioned threaded shaft, comprising a head, a threaded shaft with a spherical end, an upper thrust element, and a lower thrust element, the head of the screw is provided with a slot from the above in which a fixation rod is placed, the fixation rod is arrested with the upper thrust element also fixed in the head of the screw where the bottom of the slot is connected with the outer side of the head of the screw through an opening that leads out eccentrically at the transition of the base and the outer side of the head of the screw, the upper surface of the lower thrust element is in contact with the fixation rod and the lower surface of the lower thrust element is in contact with the spherical end of the threaded shaft, according to the invention whose principle lies in the fact that in the opening the lower thrust element and the spherical end of the threaded shaft are placed, and the lower thrust element presses the spherical end of the threaded shaft to the wall of the opening in the head of the screw.

It is advantageous, if the upper thrust element is fixed in the threaded walls of the head of the screw (i.e. screwed in the head of the screw) and if the lower thrust element is movably connected with the head of the screw using a hinge placed at the bottom of the slot in the head of the screw on the side opposite to the opening in the head of the screw. It is also advantageous if the shaft of the hinge is removable. This facilitates quick assembly of the whole set of the transpedicular screw. The fixation rod arrested in the slot in the head of the screw presses with the upper thrust element to the upper surface of the lower thrust element. Thus, the lower surface of the lower thrust element presses to the spherical end of the threaded shaft against the wall of the opening in the head of the screw causing the threaded shaft to be arrested in the required position against the head of the screw.

For better stability of the seating of the lower thrust element in the head of the screw, straight or arched grooves can be arranged on the longer sides of the lower thrust element which would fit into the prominences in the opening in the head of the screw. The straight or the arched grooves can alternatively be arranged in the opening in the head of the screw on the understanding that the prominences on the longer sides of the lower thrust element would fit into them.

Higher strength of the arrestment of the spherical end of the threaded shaft in the head of the screw can be achieved if the lower thrust element is provided with a spherical hollow on its lower surface for the spherical end of the threaded shaft or, possibly, if the contact surfaces of the lower thrust element and the spherical end of the threaded shaft are roughened or provided with teeth.

For easier and quicker introduction of the threaded shaft into the vertebral body and for easier and quicker arrestment of the fixation rod in the head of the screw, it is advantageous for the upper thrust element and the spherical end of the threaded shaft to be provided with holes for an assembly instrument on their upper surfaces.

It is also advantageous if the opening in the head of the screw is provided with a spherical hollow which is in contact with the spherical end of the threaded shaft.

It is also advantageous if the lower thrust element is movably connected with the head of the screw using a connecting component freely arranged in the opening in the lower thrust element and fixed in the screw head base.

Owing to the fact that the threaded shaft in the head of the screw is mounted asymmetrically, the polyaxial screw with the eccentrically mounted threaded shaft combines the advantages of easy preoperative handling (identical for all systems with polyaxial screws) with the chance of preventing the surfaces of the facet intervertebral joint from being damaged by the contact between the facet intervertebral joint and the head of the screw, as the head of the polyaxial screw with eccentrically mounted threaded shaft is (due to this particular design) seated out of the facet joint.

### Overview of the Figures

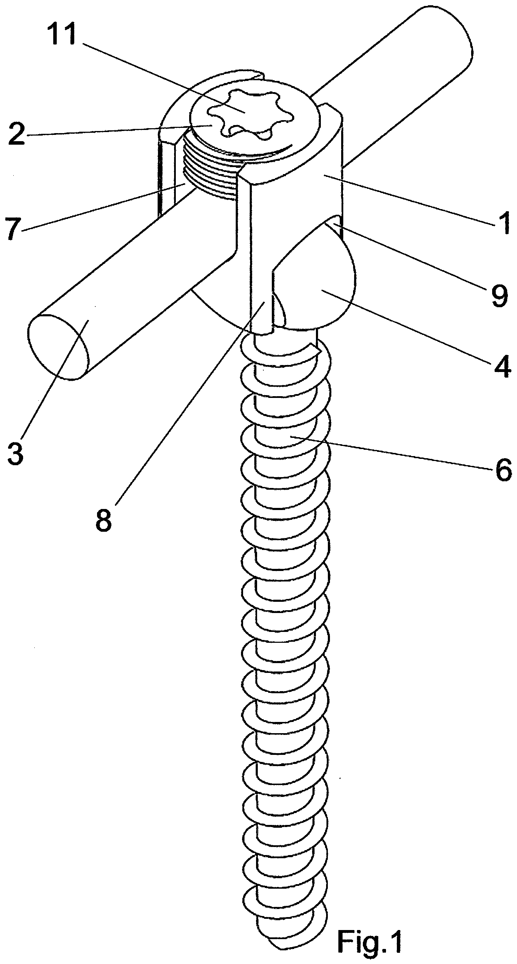

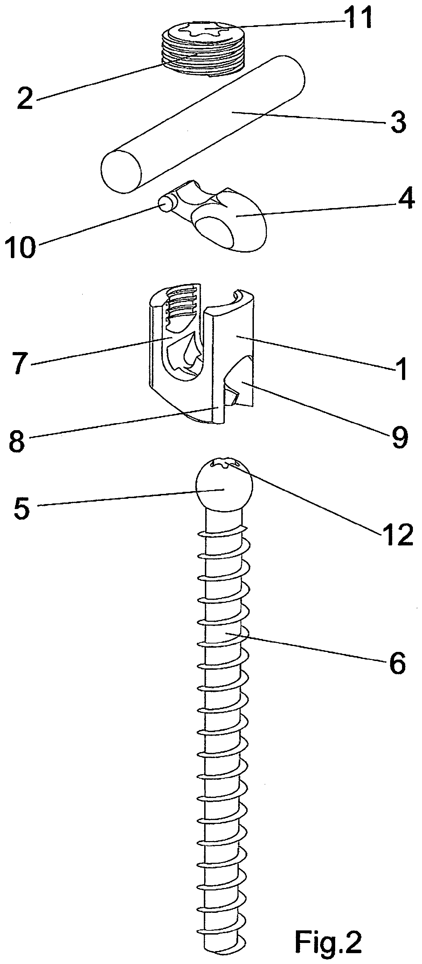

The nature of the invention is better clarified in the drawings. Fig. 1 shows an assembled axonometric view of the general arrangement of the Transpedicular Polyaxial Screw with smooth contact surfaces. Fig. 2 shows an expanded axonometric view of the general arrangement of the Transpedicular Polyaxial Screw.

### Example of the Performance of the Invention

The Transpedicular polyaxial screw (Fig. 1, Fig. 2) with eccentrically mounted threaded shaft consists of the head 1 of the screw and the threaded shaft of the screw 6 with the spherical end 5. The head 1 of the screw is provided with a slot 7 on its upper surface in which the fixation rod 3 is seated. The fixation rod 3 is arrested with the upper thrust element 2 also fixed in the head 1 of the screw. The bottom of the slot 7 is connected with the outer side 8 of the head 1 of the screw through an opening 9 that leads out eccentrically at the transition of the base and the outer side 8 of the head 1 of the screw. In the opening, the lower thrust element 4 and the spherical end 5 of the threaded shaft 6 are placed. The upper surface of the lower thrust element 4 is in contact with the fixation rod 2 and the lower surface of the lower thrust element 4 is in contact with the spherical end 5 of the threaded shaft 6 pressing it to the wall of the opening 9 in the head 1 of the screw.

The upper thrust element 2 is fixed by thread in the head 1 of the screw. The lower thrust element 4 is movably connected with the head 1 of the screw using a hinge 10 placed at the bottom of the slot 7 in the head 1 of the screw on the side opposite to the opening 9 in the head 1 of the screw. The shaft of the hinge 10, for the movable connection between the head 1 of the screw and the lower thrust element 4, is removable.

Straight or arched grooves are arranged on the longer sides of the lower thrust element 4 which fit into the prominences in the opening 9 of the head 1 of the screw.

The straight or the arched grooves can alternatively be arranged in the opening 9 of the head 1 of the screw which the prominences on the longer sides of the lower thrust element 4 fit into.

The lower surface of the lower thrust element 4 is provided with a spherical hollow on its lower surface for the spherical end 5 of the threaded shaft 6.

The opening 9 in the head 1 of the screw is provided with a spherical hollow which is in contact with the spherical end 5 of the threaded shaft 6.

The upper surface of the upper thrust element 2 is provided with a hole 11 for an assembly instrument. The upper surface of the spherical end 5 of the threaded shaft 6 is provided with a hole 12 for an assembly instrument.

The surface of the spherical end 5 of the threaded shaft 6 and/or the surface of the spherical hollow in the lower thrust element 4 are/is roughened or provided with teeth.

The lower thrust element 4 is movably connected with the head 1 of the screw using a connecting component freely arranged in the opening in the lower thrust element 4 and fixed in the base of the head 1 of the screw.

### Industrial Application

The Transpedicular polyaxial screw with the eccentrically mounted threaded shaft is intended to be used in the field of spine surgery.

### List of Reference Marks

- 1: Head of the screw
- 2: Upper thrust element
- 3: Fixation rod
- 4: Lower thrust element
- 5: Spherical end
- 6: Threaded shaft
- 7: Slot
- 8: Outer side
- 9: Opening
- 10: Hinge
- 11: Hole for an assembly instrument I
- 12: Hole for an assembly instrument II

## Claims

1. A transpedicular polyaxial screw, especially a transpedicular polyaxial screw with eccentrically mounted threaded shaft, comprising a head, a threaded shaft with a spherical end, an upper thrust element, and a lower thrust element, the head (1) of the screw is provided with a slot (7) from the above in which a fixation rod (3) is placed, the fixation rod (3) is arrested with the upper thrust element (2) also fixed in the head (1) of the screw where the bottom of the slot (7) is connected with the outer side (8) of the head (1) of the screw through an opening (9) that leads out eccentrically at the transition of the base and the outer side (8) of the head (1) of the screw, the upper surface of the lower thrust element (4) is in contact with the fixation rod (2) and the lower surface of the lower thrust element (4) is in contact with the spherical end (5) of the threaded shaft (6), **distinguishing itself** by the fact that in the opening (9) the lower thrust element (4) and the spherical end (5) of the threaded shaft (6) are placed, and the lower thrust element (4) presses the spherical end (5) of the threaded shaft (6) to the wall of the opening (9) in the head (1) of the screw.

2. The transpedicular polyaxial screw according to claim 1 **distinguishing itself** by the upper thrust element (2) being screwed in the head (1) of the screw.

3. The transpedicular polyaxial screw according to any of the previous claims **distinguishing itself** by the fact that the lower thrust element (4) is movably connected with the head (1) of the screw using a hinge (10) placed at the bottom of the slot (7) in the head (1) of the screw on the side opposite to the opening (9) in the head (1) of the screw.

4. The transpedicular polyaxial screw according to claim 3 **distinguishing itself** by the fact that the shaft of the hinge (10) is removable.

5. The transpedicular polyaxial screw according to any of the previous claims **distinguishing itself** by the fact that the lower thrust element (4) has longer sides, where are arranged straight or arched grooves, which fit into prominences in an opening (9) in the head (1) of the screw.

6. The transpedicular polyaxial screw according to any of the previous claims 1 through 4 **distinguishing itself** by the fact that in an opening (9) in the head (1) are arranged straight or arched grooves in which fit prominences on the longer sides of the lower thrust element (4).

7. The transpedicular polyaxial screw according to any of the previous claims **distinguishing itself** by the fact that the lower thrust element (4) is provided with a spherical hollow on its lower surface for the spherical end (5) of the threaded shaft (6).

8. The transpedicular polyaxial screw according to any of the previous claims **distinguishing itself** by the fact that the opening (9) in the head (1) of the screw is provided with a spherical hollow which is in contact with the spherical end (5) of the threaded shaft (6).

9. The transpedicular polyaxial screw according to any of the previous claims **distinguishing itself** by the fact that the upper surface of the upper thrust element (2) is provided with a hole (11) for an assembly instrument.

10. The transpedicular polyaxial screw according to any of the previous claims **distinguishing itself** by the fact that the upper surface of the spherical end (5) of the threaded shaft (6) is provided with a hole (12) for an assembly instrument.

11. The transpedicular polyaxial screw according to any of the previous claims **distinguishing itself** by the fact that the surface of the spherical end (5) of the threaded shaft (6) and/or the surface of the spherical hollow in the lower thrust element (4) is/are roughened or provided with teeth.

12. The transpedicular polyaxial screw according to any of the previous claims **distinguishing itself** by the fact that the lower thrust element (4) is movably connected with the head (1) of the screw using a connecting component freely arranged in the opening in the lower thrust element (4) and fixed in the base of the head (1) of the screw.

## Patentansprüche

1. Die transpedikuläre Polyaxialschraube, insbesondere die transpedikuläre Polyaxialschraube mit der exzentrisch gelagerten Gewindewelle enthält den Kopfteil, die Gewindewelle mit dem Kugelansatz, das obere und untere Druckelement, der Schraubenkopf (1) ist oben mit einem Schlitz (7) versehen, in dem ein Fixierstab (3) eingelagert ist, der Fixierstab (3) wird mit dem oberen Druckelement (2) arretiert, welches zugleich in dem Schraubenkopf (1) befestigt wird, wo der Schlitzboden (7) mit der Außenwand (8) des Schraubenkopfes (1) durch die Öffnung (9) verbunden ist, die am Übergang der Basis und der Außenseite (8) des Schraubenkopfes (1) exzentrisch mündet, wobei das untere Druckelement (4) mit dessen Oberfläche mit dem Fixierstab (2) und der unteren Fläche mit einem kugelförmigen Ende (5) der Gewindewelle (6) im Kontakt steht, ist **dadurch gekennzeichnet, dass** in der Öffnung (9) das untere Druckelement (4) und der Kugelansatz (5) der Gewindewelle (6) untergebracht sind, und das untere Druckelement (4) den Kugelansatz (5) der Gewindewelle (6) an die Wand der Öffnung (9) im Kopfteil (1) der Schraube drückt.

2. Die transpedikuläre Polyaxialschraube, nach der Anforderung 1, ist **dadurch gekennzeichnet, dass** das obere Druckelement (2) mit dessen Gewinde im Kopfteil (1) der Schraube befestigt ist.

3. Die transpedikuläre Polyaxialschraube, nach einer der vorherigen Anforderungen, ist **dadurch gekennzeichnet, dass** das untere Druckelement (4) mit dem Kopfteil (1) der Schraube durch ein Scharnier (10) auf dem Schlitzboden (7) des Kopfteils (1) der Schraube auf der entgegengesetzten Seite der Öffnung (9) im Kopfteil (1) der Schraube gegenüber flexibel verbunden ist.

4. Die transpedikuläre Polyaxialschraube, nach der Anforderung 3, ist **dadurch gekennzeichnet, dass** die Welle des Scharniers (10) abnehmbar ist.

5. Die transpedikuläre Polyaxialschraube, nach einer der vorherigen Anforderungen, ist **dadurch gekennzeichnet, dass** das untere Druckelement (4) längere Seiten aufweist, auf denen gerade oder Bogenrillen angebracht sind, die in die Ansätze der Öffnung (9) des Kopfteils (1) der Schraube einschnappen.

6. Die transpedikuläre Polyaxialschraube, nach einer der Anforderungen 1 bis 4, ist **dadurch gekennzeichnet dass** in der Öffnung (9) des Kopfteiles (1) der Schraube gerade oder Bogenrillen angebracht sind, in die die Ansätze auf längeren Seiten des Druckelements (4) einschnappen.

7. Die transpedikuläre Polyaxialschraube, nach einer der vorherigen Anforderungen, ist **dadurch gekennzeichnet, dass** das untere Druckelement (4) auf dessen unteren Fläche eine kugelförmige Vertiefung für den Kugelansatz (5) der Gewindewelle (6) hat.

8. Die transpedikuläre Polyaxialschraube, nach einer der vorherigen Anforderungen, ist **dadurch gekennzeichnet, dass** sich in der Öffnung (9) im Kopfteil (1) der Schraube eine Vertiefung mit dem Kugelansatz befindet, mit dem kugelförmigen Ansatz (5) der Gewindewelle (6) im Kontakt steht.

9. Die transpedikuläre Polyaxialschraube, nach einer der vorherigen Anforderungen, ist **dadurch gekennzeichnet, dass** das obere Druckelement (2) auf dessen oberen Fläche mit einer Öffnung (11) für ein Montagewerkzeug versehen ist.

10. Die transpedikuläre Polyaxialschraube, nach einer der vorherigen Anforderungen, ist **dadurch gekennzeichnet, dass** der kugelförmige Ansatz (5) der Gewindewelle (6) auf dessen oberen Fläche mit einer Öffnung (12) für ein Montagewerkzeug versehen ist.

11. Die transpedikuläre Polyaxialschraube, nach einer der vorherigen Anforderungen ist **dadurch gekennzeichnet, dass** der kugelförmige Ansatz (5) der Gewindewelle (6) und/oder die kugelförmige Vertiefung des unteren Druckelements (4) auf deren Oberfläche mit Aufrauung oder Verzahnung versehen sind.

12. Die transpedikuläre Polyaxialschraube, nach einer der vorherigen Anforderungen, ist **dadurch gekennzeichnet, dass** das untere Druckelement (4) mit dem Kopfteil (1) der Schraube durch ein in der Öffnung des unteren Druckelements (4) frei gelagertes und in der Basis des Kopfteils (1) der Schraube befestigten Verbindungselement flexibel verbunden ist.

## Revendications

1. La vis transpediculaire polyaxiale, notamment la vis transpediculaire polyaxiale avec l'arbre à filetage placé de façon excentrique, comporte la tête, l'arbre à filetage avec le bout sphérique, l'élément de pression supérieur et l'élément de pression inférieur, à la tête (1) de la vis, il y a une entaille (7) d'en haut, dans laquelle se trouve la barre de fixation (3), la barre de fixation (3) est arrêtée par l'élément de pression supérieur (2), qui est en même temps fixé dans la tête (1) de la vis, où le fond de l'entaille (7) est joint à la face externe (8) de la tête (1) de la vis par une ouverture (9) qui débouche de façon excentrique au passage de la base et de la face externe (8) de la tête (1) de la vis, et en même temps l'élément de pression inférieur (4) est par sa surface supérieure au contact avec la barre de fixation (2) et par la surface inférieure avec le bout sphérique (5) de l'arbre à filetage (6) **caractérisé en ce que** dans l'ouverture (9) il y a l'élément de pression inférieur (4) et le bout sphérique (5) de l'arbre à filetage (6), et l'élément de pression inférieur (4) fait la pression au bout sphérique (5) de l'arbre à filetage (6) contre la paroi de l'ouverture (9) dans la tête (1) de la vis.

2. La vis transpediculaire polyaxiale, selon la revendication 1 **caractérisé en ce que** l'élément de pression supérieur (2) est fixé par le filetage dans la tête (1) de la vis.

3. La vis transpediculaire polyaxiale, selon une des revendications précédentes, **caractérisé en ce que** l'élément de pression inférieur (4) est de façon mobile joint à la tête (1) de la vis par un gond (10) placé au fond de l'entaille (7) de la tête (1 ) de la vis à l'opposé contre l'ouverture (9) dans la tête (1) de la vis.

4. La vis transpediculaire polyaxiale, selon la revendication 3, **caractérisé en ce que** l'arbre du gond (10) est amovible.

5. La vis transpediculaire polyaxiale, selon une des revendications précédentes, **caractérisé en ce que** l'élément de pression inférieur (4) a les côtés plus longues auxquelles sont rangés des rainures droits ou en arc entrant dans les encoches de l'ouverture (9) de la tête (1) de la vis.

6. La vis transpediculaire polyaxiale, selon une des revendications précédentes 1 jusqu'à 4, **caractérisé en ce que** dans l'ouverture (9) de la tête (1) de la vis sont rangés des rainures droits ou en arc entrant dans les encoches dans lesquelles entrent les encoches aux côtés plus longues de l'élément de pression inférieur (4).

7. La vis transpediculaire polyaxiale, selon une des revendications précédentes, **caractérisé en ce que** l'élément de pression inférieur (4) a, à sa surface inférieure, un creux sphérique pour la terminaison sphérique (5) de l'arbre à filetage (6).

8. La vis transpediculaire polyaxiale, selon une des revendications précédentes, **caractérisé en ce que** dans l'ouverture (9) de la tête (1) de la vis est en creux à surface sphérique qui est au contact à la terminaison sphérique (5) de l'arbre à filetage (6).

9. La vis transpediculaire polyaxiale, selon une des revendications précédentes, **caractérisé en ce que** l'élément de pression supérieur (2) est pourvu sur sa surface supérieure par l'ouverture (11) pour l'outil de montage.

10. La vis transpediculaire polyaxiale, selon une des revendications précédentes, **caractérisé en ce que** la terminaison sphérique (5) de l'arbre à filetage (6) la terminaison sphérique (5) de l'arbre à filetage (6) est pourvu sur sa surface supérieure par l'ouverture (12) pour l'outil de montage.

11. La vis transpediculaire polyaxiale, selon une des revendications précédentes, **caractérisé en ce que** la terminaison sphérique (5) de l'arbre à filetage (6) et/ou le creux de l'élément de pression inférieur (4) sont rendus rugueux sur la surface ou pourvus de la denture.

12. La vis transpediculaire polyaxiale, selon une des revendications précédentes, **caractérisé en ce que** l'élément de pression inférieur (4) est joint à la tête (1) de la vis de façon mobile par un élément de jonction placé librement dans l'ouverture de l'élément de pression inférieur (4) et fixé dans la base de la tête (1) de la vis.
